# EUROPEAN PATENT SPECIFICATION

(11) **EP 2 336 665 B1**
(45) Date of publication and mention of the grant of the patent: **16.05.2018**
(21) Application number: 09812865.5
(22) Date of filing: 04.09.2009
(51) Int. Cl.: F24F 13/20, A61L 9/14, F24F 1/00, F24F 6/00, F24F 6/12, F24F 3/12

(54) **AIR CONDITIONER**
KLIMAANLAGE
CLIMATISEUR

(30) Priority: 12.09.2008 JP 2008234698; 28.08.2009 JP 2009198393
(43) Date of publication of application: 22.06.2011
(73) Proprietor: Panasonic Corporation, Kadoma-shi Osaka 571-8501 (JP)
(72) Inventor: YAMAGUCHI, Narito, Osaka-shi, Osaka 5406207 (JP); EBIHARA, Masaharu, Osaka-shi, Osaka 5406207 (JP); TAKAHASHI, Masatoshi, Osaka-shi, Osaka 5406207 (JP); KAWAZOE, Daisuke, Osaka-shi, Osaka 5406207 (JP); YONEZAWA, Masaru, Osaka-shi, Osaka 5406207 (JP); BAMBA, Masahiro, Osaka-shi, Osaka 5406207 (JP); NAKAGAWA, Hideaki, Osaka-shi, Osaka 5406207 (JP); MEKATA, Masato, Osaka-shi, Osaka 5406207 (JP); DAIMON, Hiroyuki, Osaka-shi, Osaka 5406207 (JP); AKIYAMA, Jun, Osaka-shi, Osaka 5406207 (JP)
(74) Representative: Eisenführ Speiser
(86) International application number: PCT/JP2009/004387
(87) International publication number: WO 2010/029716

(56) References cited:
- WO-A1-2008/065737
- JP-A- 2006 029 663
- JP-A- 2008 010 820
- JP-A- 2008 029 973
- JP-A- 2008 133 980
- JP-A- 2008 177 314
- JP-A- 2008 190 811
- JP-A- 2008 190 813

## Description

### Technical Field

The present invention relates to an air conditioner provided with an indoor unit that has an air cleaning function for cleaning indoor air.

### Background Art

Some conventional air conditioners have a deodorizing function. For this purpose, they include an air cleaning filter disposed in proximity to, for example, suction openings in an indoor unit to adsorb odorous components or a deodorizing unit disposed in, for example, an air passage and having an oxidation decomposing function to adsorb the odorous components.

However, because the air conditioners having a deodorizing function remove odorous components contained in air, which has been sucked through the suction openings, for deodorization, they cannot remove odorous components contained in air inside a room or those adhering to curtains, walls, and the like.

In view of this, an air conditioner having an electrostatic atomizing device has been proposed. The electrostatic atomizing device is disposed in the air passage in the indoor unit to generate an electrostatic mist of a nanometer size in particle diameter and subsequently blow out it together with air into the room, thereby removing the odorous components contained in indoor air or those adhering to curtains, walls, and the like (see, for example, Patent Document 1 or 2).

In such an air conditioner, the electrostatic atomizing device is disposed in proximity to, for example, the suction openings or a discharge opening, or downstream of a heat exchanger or an indoor fan.
[Patent Document 1] Japanese Laid-Open Patent Publication No. 2005-282873
[Patent Document 2] Japanese Laid-Open Patent Publication No. 2006-234245

WO2008065737A1 discloses an air conditioner having a function of carrying a mist of charged minute water particles on a ventilated air being blown into a room. The air conditioner includes a blower blowing the ventilated air and an atomizing unit configured to be supplied with water and to electrostatically atomize it into the mist of minute charged water particles. A housing of the air conditioner is divided by a partition into a flow channel leading from an air inlet to an air outlet and a compartment accommodating therein the atomizing unit. A feed conduit extends from a feed port of the atomizing unit into a flow channel through the partition to feed the mist to the air being blown out through the air outlet, while the atomizing unit is isolated from the flow channel to minimize flow resistance.

### Summary of the Invention

### Problems to be Solved by the Invention

However, the air conditioner as disclosed in Patent Document 1 or 2 employs a Peltier element for condensation of moisture contained in air, and the Peltier element is cooled by air in the air passage. Accordingly, the Peltier element can be cooled during cooling, but cannot be cooled during heating. That is, Patent Document 1 or 2 does not provide any specific proposal as to the cooling of the Peltier element that is an issue in practical use.

The present invention has been developed to overcome the above-described disadvantage.

It is accordingly an objective of the present invention to provide an air conditioner capable of generating an electrostatic mist by efficiently cooling the Peltier element.

### Means to Solve the Problems

In accomplishing the above objective, the air conditioner according to the present invention has an indoor unit that includes a heat exchanger, an indoor fan operable to convey air heat exchanged by the heat exchanger, and a discharge opening defined therein, through which the air conveyed by the indoor fan is blown out, the air conditioner including a partition wall that partitions an inside of the indoor unit into first and second compartments with the heat exchanger and the indoor fan accommodated within the first compartment. The first compartment defines a main passage through which the air conveyed by the indoor fan is conveyed before the air is blown out through the discharge opening. The second compartment acts as a bypass passage for bypassing the heat exchanger and the indoor fan. An electrostatic atomizing unit is accommodated within the second compartment and operable to generate dew condensation using moisture contained in air, then generate an electrostatic mist by high voltage discharge, and discharge the electrostatic mist into a room. The electrostatic atomizing unit includes a Peltier element operable to generate dew condensation water by cooling, a discharge electrode operable to cause the high
voltage discharge to generate the electrostatic mist from the dew condensation water, and a radiating portion operable to dissipate heat from the Peltier element and including a plurality of fins with air gaps defined therebetween. The partition wall has an opening defined therein through which the first and second compartments communicate with each other. Air inside the second compartment is introduced into the first compartment through the opening in the partition wall by the action of a negative pressure inside the main passage caused by operation of the indoor fan, and the air gaps between the fins are formed substantially along an air flow introduced from the second compartment into the first compartment. By this construction, an air flow is generated in the second compartment, in which the electrostatic atomizing unit is installed, by the action of the indoor fan, and the air flows smoothly along the air gaps between the fins to efficiently cool the Peltier element.

### Effects of the Invention

The air conditioner according to the present invention can positively and efficiently cool the Peltier element of the electrostatic atomizing unit to thereby keep the electrostatic atomizing unit generating the electrostatic mist positively and steadily for a long period of time.

### Brief Description of the Drawings

Fig. 1 is a perspective view of an indoor unit of an air conditioner according to the present invention with a portion thereof removed.
Fig. 2 is a schematic vertical sectional view of the indoor unit of Fig. 1.
Fig. 3 is a perspective view of an electrostatic atomizing device mounted in the indoor unit of Fig. 1.
Fig. 4 is a front view of the electrostatic atomizing device and a portion of a frame of the indoor unit of Fig. 1.
Fig. 5 is a schematic view of the electrostatic atomizing device.
Fig. 6 is a block diagram of the electrostatic atomizing device.
Fig. 7 is a perspective view of the indoor unit, particularly depicting a state in which the electrostatic atomizing device is mounted with respect to a body of the indoor unit.
Fig. 8 is a perspective view of a modification of the indoor unit, particularly depicting a state in which the electrostatic atomizing device is mounted with respect to the body of the indoor unit.
Fig. 9 is a side view of the indoor unit of Fig. 1, particularly depicting a positional relationship between the electrostatic atomizing device and a ventilation fan unit.
Fig. 10 is a perspective view of an automatic filter cleaning device mounted in the indoor unit of Fig. 1.
Fig. 11 is a perspective view of a modification of the electrostatic atomizing device.
Fig. 12 is a side view of the indoor unit of Fig. 1, particularly depicting a positional relationship between the electrostatic atomizing device of Fig. 11 and the ventilation fan unit.
Fig. 13 is a front view of an end portion of the indoor unit of Fig. 12 with a portion thereof removed.
Fig. 14 is a perspective view showing a state before the electrostatic atomizing unit secured to a suction device is mounted on the frame.
Fig. 15 is a front view of an end portion of the indoor unit before the electrostatic atomizing unit is mounted on the frame.
Fig. 16 is a side view of the indoor unit, partly in section, showing a state before the electrostatic atomizing unit is mounted on the frame.

### Embodiments of the Invention

In an aspect of the present invention, an air conditioner has an indoor unit that includes a heat exchanger, an indoor fan operable to convey air heat exchanged by the heat exchanger, and a discharge opening defined therein, through which the air conveyed by the indoor fan is blown out, the air conditioner including a partition wall that partitions an inside of the indoor unit into first and second compartments with the heat exchanger and the indoor fan accommodated within the first compartment. The first compartment defines a main passage through which the air conveyed by the indoor fan is conveyed before the air is blown out through the discharge opening. The partition wall has an opening defined therein through which the first and second compartments communicate with each other. An electrostatic atomizing unit is accommodated within the second compartment and operable to generate dew condensation using moisture contained in air, then generate an electrostatic mist by high voltage discharge, and discharge the electrostatic mist. The electrostatic atomizing unit includes a Peltier element operable to generate dew condensation water by cooling, a discharge electrode operable to cause the high voltage discharge to generate the electrostatic mist from the dew condensation water, and a radiating portion operable to dissipate heat from the Peltier element and including a plurality of fins with air gaps defined therebetween. By this construction, operation of the indoor fan generates a negative pressure inside the main passage to thereby introduce air inside the second compartment into the first compartment through the opening in the partition wall. The air gaps between the plurality of fins are formed substantially in a direction in which the air introduced from the second compartment into the first compartment flows. An air flow is generated in the second compartment, in which the electrostatic atomizing unit is installed, by the action of the indoor fan, and the air flows smoothly along the air gaps between the fins to efficiently cool the Peltier element, thus making it possible to keep the electrostatic atomizing unit generating the electrostatic mist positively and efficiently.

The opening in the partition wall is positioned adjacent the discharge opening, and operation of the indoor fan renders the main passage to be a negative pressure portion of a pressure lower than that of the second compartment. By so doing, the electrostatic mist generated in the second compartment can be positively discharged into a room through the discharge opening.

The opening in the partition wall is positioned upstream of the indoor fan, and operation of the indoor fan renders the main passage to be a negative pressure portion of a pressure lower than that of the second compartment. By so doing, the electrostatic mist generated in the second compartment can be positively discharged into a room through the discharge opening.

The fins of the radiating portion are highest at a central portion confronting the Peltier element and become lower toward outside. By this configuration, the centrally-located fins are cooled by fresh air due to a difference in the height of the fins in addition to ascending currents in the air gaps, thus making it possible to further enhance the cooling effect of the Peltier element and increase the amount of the electrostatic mist.

The indoor unit has an opening defined in a portion thereof for communication with an outside of the indoor unit such that the opening confronts the air gaps between the fins. This configuration allows air outside the indoor unit to flow through the air gaps, thereby further enhancing the cooling effect of the Peltier element and increasing the amount of the electrostatic mist.

Embodiments of the present invention are explained hereinafter with reference to the drawings, but the present invention is not limited by the embodiments.

### Embodiment 1.

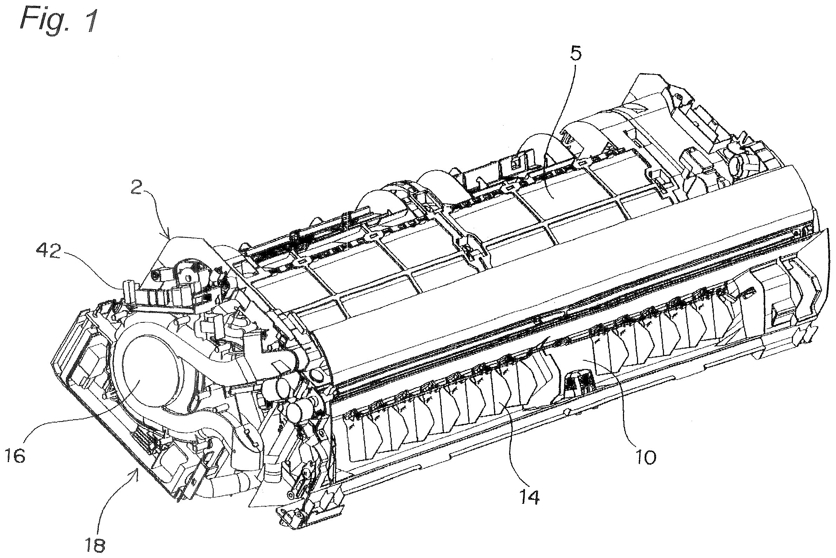

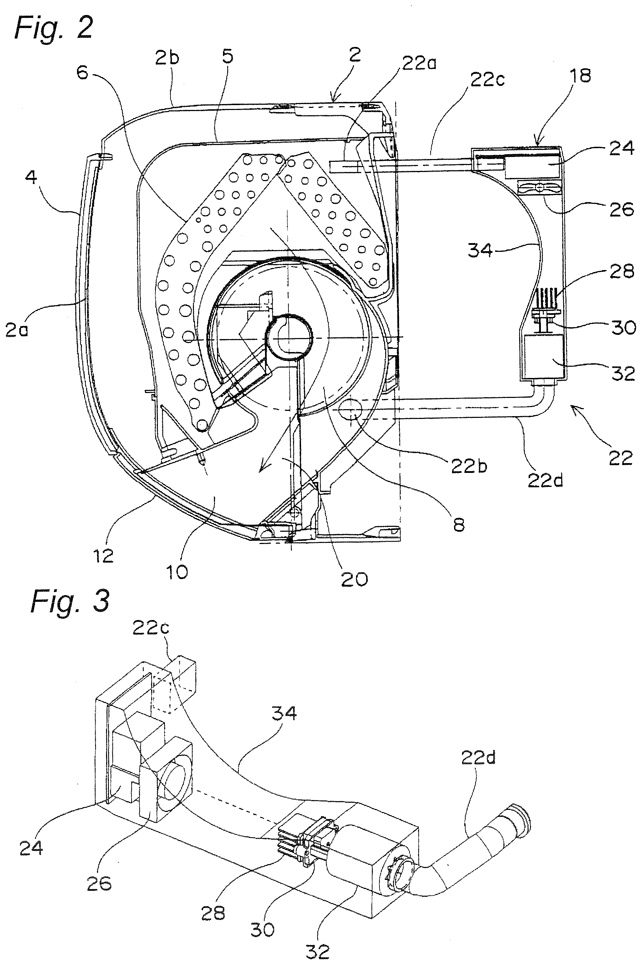

An air conditioner includes an outdoor unit and an indoor unit connected to each other via refrigerant piping, and Figs. 1 and 2 depict the indoor unit of the air conditioner according to the present invention.

As shown in Figs. 1 and 2, the indoor unit includes a main body 2 having front suction openings 2a and upper suction openings 2b both defined therein as suction openings through which indoor air is sucked into the main body 2. The indoor unit also includes a movable front panel (hereinafter referred to simply as "front panel") 4 to open and close the front suction openings 2a. When the air conditioner is not in operation, the front panel 4 is held in close contact with the main body 2 to close the front suction openings 2a, while when the air conditioner is brought into operation, the front panel 4 moves away from the main body 2 to open the front suction openings 2a.

The main body 2 accommodates therein a filter 5 disposed downstream of the front suction openings 2a and the upper suction openings 2b to remove dust contained in air, a heat exchanger 6 disposed downstream of the filter 5 to heat exchange with indoor air sucked through the front suction openings 2a and the upper suction openings 2b, a fan (hereinafter referred to as "indoor fan") 8 operable to convey air heat exchanged by the heat exchanger 6, vertical air direction changing blades 12 operable to open and close a discharge opening 10, through which air conveyed by the indoor fan 8 is blown out into a room, and also operable to vertically change the direction of air blown out from the discharge opening 10, and horizontal air direction changing blades 14 operable to horizontally change the air direction. The front panel 4 is connected at an upper portion thereof to an upper portion of the main body 2 via a plurality of arms (not shown) provided on respective side portions thereof. Because one of the plurality of arms is connected to a drive motor (not shown), when the air conditioner is brought into operation, the front panel 4 is moved forward from a position (where the front suction openings 2a are closed) during a halt of the air conditioner by driving the drive motor. The vertical air direction changing blades 12 are similarly connected to a lower portion of the main body 2 via a plurality of arms (not shown) provided on respective side portions thereof.

A ventilation fan unit 16 operable to ventilate indoor air is disposed at a side portion (at a left side portion as viewed from the front of the indoor unit or on a side of a bypass passage 22 with respect to a partition wall 46c explained later) of the indoor unit, and an electrostatic atomizing device 18 is disposed rearwardly of the ventilation fan unit 16. The electrostatic atomizing device 18 has an air cleaning function of cleaning indoor air by generating an electrostatic mist.

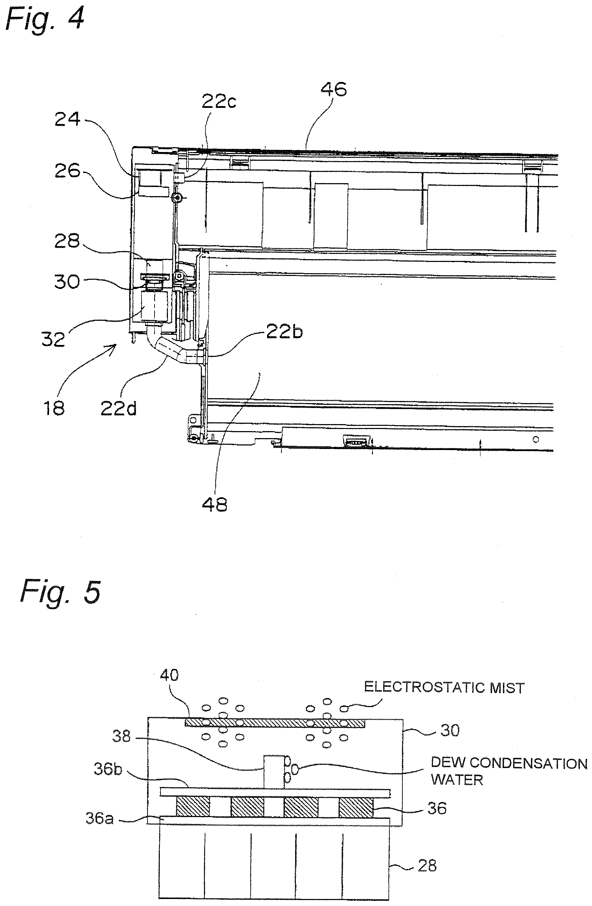

It is to be noted here that Fig. 1 depicts a state in which the front panel 4 and a cover (not shown) for covering the main body 2 are both removed, and Fig. 2 depicts a state in which the electrostatic atomizing device 18 accommodated within the main body 2 is separated from the main body 2 for clarification of junctions between the main body 2 of the indoor unit and the electrostatic atomizing device 18. In fact, the electrostatic atomizing device 18 has a shape as shown in Fig. 3 and is mounted in a left side portion of the main body 2, as shown in Fig. 1 or 4.

As shown in Figs. 2 and 4 and Fig. 7 referred to later, the inside of the main body 2 of the indoor unit is partitioned into a first compartment and a second compartment by a partition wall explained later. The heat exchanger 6, the indoor fan 8, and the like are accommodated within the first compartment, which constitutes a main passage 20 that communicates the front suction openings 2a and the upper suction openings 2b with the discharge opening 10 via the heat exchanger 6, the indoor fan 8, and the like. On the other hand, the second compartment is a space 22 accommodating the electrostatic atomizing device 18 therein. Because this space 22 acts as a bypass passage for bypassing the heat exchanger 6 and the indoor fan 8, the space 22 is hereinafter referred to as the bypass passage. A high voltage transformer 24 employed as a high voltage power source and a bypass fan 26 are disposed on an upstream side of the bypass passage 22, and an electrostatic atomizing unit 30 having a radiating portion 28 to promote radiation thereof and a silencer 32 are disposed on a downstream side of the bypass passage 22. Accordingly, the high voltage transformer 24, the bypass fan 26, the radiating portion 28, the electrostatic atomizing unit 30, and the silencer 32 are arrayed in this order from the upstream side, and all of them are accommodated within a casing 34 constituting a portion of the bypass passage 22, thus making it possible to enhance assemblage. Also, because the casing 34 forms the passage, not only can a mounting space for these members be reduced, but an air flow created by the bypass fan 26 can also be positively directed to heating elements such as the high voltage transformer 24 and the radiating portion 28 to cool them. In addition, the electrostatic mist generated by the electrostatic atomizing unit 30 can be assuredly introduced to the discharge opening 10 and discharged into a room to be air conditioned.

The radiating portion 28 has a plurality of fins 28a with air gaps defined therebetween, and the fins 28a are positioned so that the air gaps may be orientated substantially along the air flow. Accordingly, air flows efficiently through the air gaps to further efficiently cool Peltier elements 36.

Further, the casing 34 is vertically arranged so that the direction of air flowing inside the casing 34 may be parallel to the direction of air flowing through the main passage 20 as viewed from the front of the main body 2 of the indoor unit. As a result, the casing 34 can be juxtaposed with the ventilation fan unit 16 in a superimposed manner as viewed from the front of the main body 2, thereby making it possible to further reduce the mounting space.

Although the high voltage transformer 24 is not always accommodated within the casing 34, it is preferred that the former be accommodated within the latter in terms of suppression of a temperature increase or reduction of the mounting space, because the high voltage transformer 24 is cooled by the air flow in the bypass passage 22.

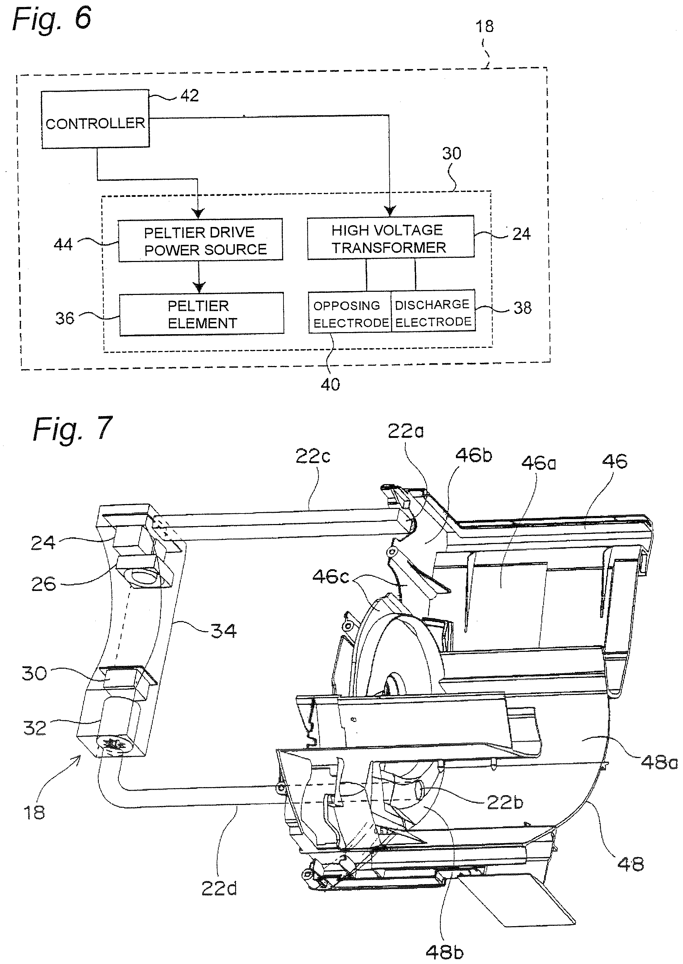

The electrostatic atomizing unit 30 is explained hereinafter with reference to Figs. 5 and 6.

As shown in Fig. 5, the electrostatic atomizing unit 30 includes a plurality of Peltier elements 36 having a radiating surface 36a and a cooling surface 36b, the radiating portion 28 referred to above and held in thermally close contact with the radiating surface 36a, a discharge electrode 38 held in thermally close contact with the cooling surface 36b via an electrical insulator (not shown) and extending upwardly from the cooling surface 36b, and an opposing electrode 40 spaced away a predetermined distance from the discharge electrode 38.

As shown in Fig. 6, the electrostatic atomizing device 18 includes a controller 42 disposed adjacent the ventilation fan unit 16 (see Fig. 1). A Peltier drive power source 44 and the high voltage transformer 24 are electrically connected to the controller 42, while the Peltier elements 36 and the discharge electrode 38 are electrically connected to the Peltier drive power source 44 and the high voltage transformer 24, respectively.

In order for the electrostatic atomizing unit 30 to generate the electrostatic mist by causing high voltage discharge from the discharge electrode 38, the opposing electrode 40 is not always required. By way of example, if one terminal of a high voltage power source is connected to the discharge electrode 38, and the other terminal of the high voltage power source is connected to the frame via a structural body, a discharge occurs between the discharge electrode 38 and a portion of the structural body connected to the frame, which portion is positioned in close proximity to the discharge electrode 38. In this case, such structural body can be regarded as the opposing electrode 40.

In the electrostatic atomizing unit 30 of the above-described construction, when the controller 42 controls the Peltier drive power source 44 to flow an electric current through the Peltier elements 36, heat is transferred from the cooling surface 36b toward the radiating surface 36a, and the temperature of the discharge electrode 38 lowers, thus resulting in dew condensation on the discharge electrode 38. Further, when the controller 42 controls the high voltage transformer 24 to apply a high voltage to the discharge electrode 38 to which dew condensation water has adhered, the dew condensation water undergoes a discharge phenomenon, which in turn generates an electrostatic mist of a nanometer size in particle diameter. Because a negative high voltage power source is employed as the high voltage transformer 24, the electrostatic mist is negatively charged.

In this embodiment, as shown in Fig. 7, the main passage 20 is delimited by a rear wall 46a of the frame 46 constituting the main body 2, opposite side walls 46b (only a left side wall is shown in Fig. 7) extending forward from respective side end portions of the rear wall 46a, a rear wall 48a of a rear guider (air guide) 48 formed below the frame 46, and opposite side walls (only a left side wall is shown in Fig. 7) 48b extending forward from respective side end portions of the rear wall 48a. One (left side wall) 46b of the side walls of the frame 46 and one (left side wall) 48b of the side walls of the rear guider 48 form a partition wall 46c that partitions the bypass passage 22 from the main passage 20. That is, the partition wall 46c separates the electrostatic atomizing device 18 provided in the bypass passage 22 from the heat exchanger 6 and the indoor fan 8. The side wall 46b of the frame 46 has a bypass suction port 22a of the bypass passage 22 defined therein, and the side wall 48b of the rear guider 48 has a bypass discharge port 22b of the bypass passage 22 defined therein.

In the case of air conditioners, low-temperature air that has passed the heat exchanger 6 of the indoor unit has a high relative humidity during cooling. Accordingly, in the electrostatic atomizing device 18 provided with the Peltier elements 36 to supply moisture, it is likely that dew condensation would occur on the whole Peltier elements 36 as well as on the discharge electrode 38 in the form of a pin. On the other hand, during heating, high-temperature air that has passed the heat exchanger 6 has a low relative humidity and, hence, the possibility of dew condensation on the discharge electrode 38 is extremely low.

In view of this, the partition wall 46c is provided to separate the bypass passage 22 from the main passage 20, and the electrostatic atomizing device 18 for generating the electrostatic mist is provided in the bypass passage 22, as described above, so that the electrostatic atomizing device 18 may be supplied with air that does not pass the heat exchanger 6 and is accordingly not controlled in temperature and humidity. By so doing, dew condensation on the whole Peltier elements 36 of the electrostatic atomizing unit 30 is effectively prevented during cooling, thereby enhancing safety, while the electrostatic mist can be positively generated even during heating.

The bypass passage 22 includes a bypass suction pipe 22c, the casing 34 referred to above, and a bypass discharge pipe 22d. One end of the bypass suction pipe 22c is connected to the bypass suction port 22a defined in the frame side wall 46b, and the bypass suction pipe 22c extends leftward from the bypass suction port 22a (in a direction generally perpendicular to the left side wall 46b and generally parallel to the front panel 4). The other end of the bypass suction pipe 22c is connected to one end of the casing 34, the other end of which is connected to one end of the bypass discharge pipe 22d that extends downward and is then bent rightward. The other end of the bypass discharge pipe 22d is connected to the bypass discharge port 22b defined in the side wall 48b of the rear guider 48. By configuring a portion of the bypass passage 22 with the casing 34, the mounting space can be reduced, and an integral configuration of these members can positively introduce the electrostatic mist from the electrostatic atomizing unit 30 into the main passage 20 via the bypass discharge pipe 22d to discharge the electrostatic mist to the room to be air conditioned.

The bypass suction port 22a is positioned between the filter 5 and the heat exchanger 6, i.e., downstream of the filter 5 and upstream of the heat exchanger 6. Because dust contained in air that has been sucked through the front suction openings 2a and the upper suction openings 2b is effectively removed by the filter 5, intrusion of the dust into the electrostatic atomizing device 18 can be suppressed, thus making it possible to effectively prevent accumulation of the dust in the electrostatic atomizing unit 30 and steadily discharge the electrostatic mist.

In this embodiment, the filter 5 serves both as a filter for the electrostatic atomizing device 18 and a filter for the main passage 20 and, hence, maintenance work is completed by cleaning only the filter 5, thus resulting in simplification of maintenance. In an air conditioner provided with an automatic filter cleaning device described later, no special maintenance work is required for the filter 5, resulting in a maintenance-free air conditioner.

On the other hand, the bypass discharge port 22b is positioned downstream of the heat exchanger 6 and the indoor fan 8 and in proximity to the discharge opening 10, so that the electrostatic mist discharged from the bypass discharge port 22b may ride an air flow in the main passage 20 and diffuse to thereby fill the whole room with the electrostatic mist. The placement of the bypass discharge port 22b at a position downstream of the heat exchanger 6 is due to the fact that if the former is positioned upstream of the latter, most (greater than about 80% to 90%) of the electrostatic mist made up of charged particles is absorbed by the heat exchanger 6 because the heat exchanger 6 is made of a metal. The placement of the bypass discharge port 22b at a position downstream of the indoor fan 8 is due to the fact that if the former is positioned upstream of the latter, because a turbulent flow is present inside the indoor fan 8, a portion (about 50%) of the electrostatic mist is absorbed by the indoor fan 8 when air flowing inside the indoor fan 8 impinges on various portions of the indoor fan 8.

Because the indoor fan 8 imparts a predetermined speed to an airflow on the side of the main passage 20 with respect to the side wall 48b of the rear guider 48 having the bypass discharge port 22b, a pressure difference is created between the side of the main passage 20 and the side of the bypass passage 22. That is, the pressure on the side of the main passage 20 is lower than that on the side of the bypass passage 22 and is accordingly negative with respect to the latter and, hence, air is introduced from the bypass passage 22 toward the main passage 20. For this reason, it is sufficient if a small-capacity fan is employed as the bypass fan 26 and, in some cases, the bypass fan 26 may be dispensed with.

The bypass discharge pipe 22d is connected to the partition wall 46c (side wall 48b of the rear guider 48) so as to extend in a direction generally perpendicular to the air flow within the main passage 20 at a junction (bypass discharge port 22b) with the main passage 20. The reason for this is that the electrostatic atomizing device 18 makes use of a discharge phenomenon to generate an electrostatic mist, as described above, and the use of the discharge phenomenon is inevitably followed by a discharge sound having a directivity. Accordingly, by connecting the bypass passage 22 to the partition wall 46c so as to extend generally parallel to the front panel 4 at the junction (bypass discharge port 22b) between the bypass passage 22 and the main passage 20, it is made extremely unlikely that the discharge sound would be directed to a person present forward or obliquely forward of the indoor unit, thereby reducing noises.

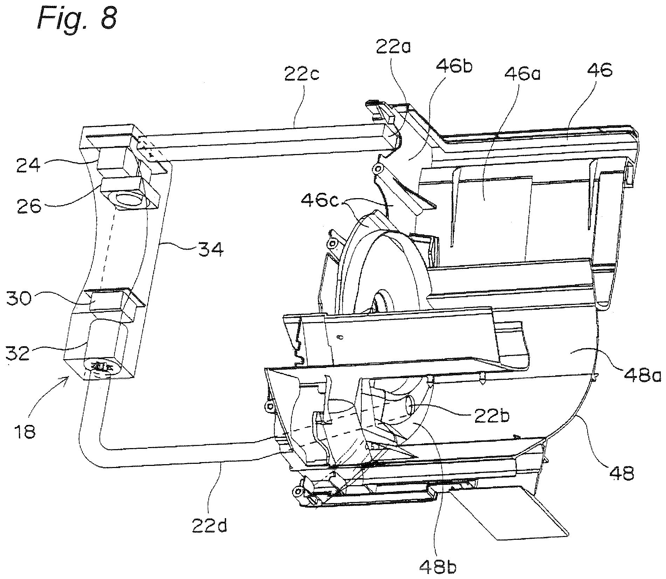

As shown in Fig. 8, the bypass discharge pipe 22d may be inclined with respect to the partition wall 46c at the junction with the main passage 20 so as to be directed upstream with respect to the air flow in the main passage 20. This configuration is effective to further reduce noises caused by the discharge sound.

Even if the bypass discharge pipe 22d is connected to the partition wall 46c so as to be directed downstream with respect to the air flow in the main passage 20, it is sufficient if an extension of the bypass discharge pipe 22d does not extend outside through the discharge opening 10. By so doing, the amount of the discharge sound emerging outside from the discharge opening 10 becomes small, and only a lesser amount of discharge sound directly reaches user's ears, thus resulting in a reduction in noise.

As described hereinabove, the main passage 20 and the bypass passage 22 are separated from each other by the partition wall 46c, and the electrostatic atomizing device 18 for generating the electrostatic mist is provided in the bypass passage 22 that bypasses the heat exchanger 6 and communicates with the main passage 20. Accordingly, the electrostatic atomizing device 18 is supplied with air that does not pass the heat exchanger 6 and is accordingly not controlled in temperature and humidity, and dew condensation on the whole Peltier elements 36 of the electrostatic atomizing unit 30 is effectively prevented during cooling, thereby enhancing safety, while the electrostatic mist can be positively generated even during heating, thus making it possible to steadily generate the electrostatic mist irrespective of the mode of operation of the air conditioner, i.e., at any time of year.

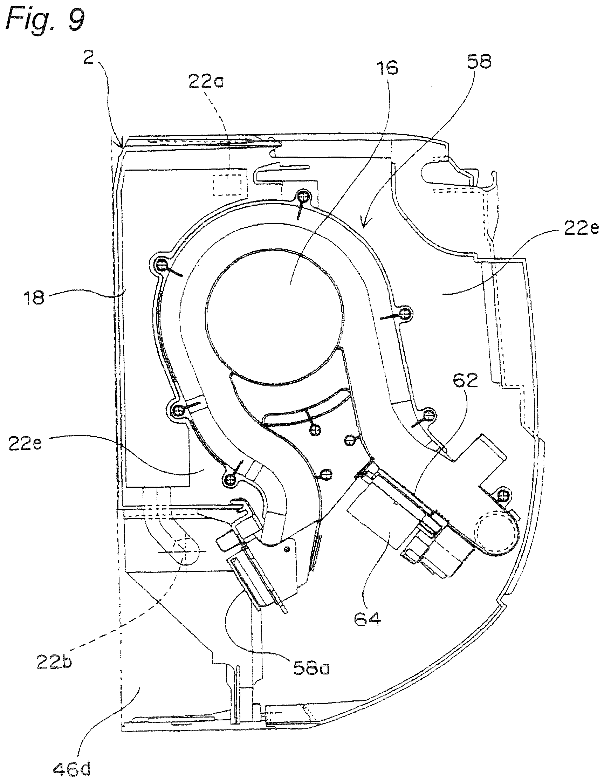

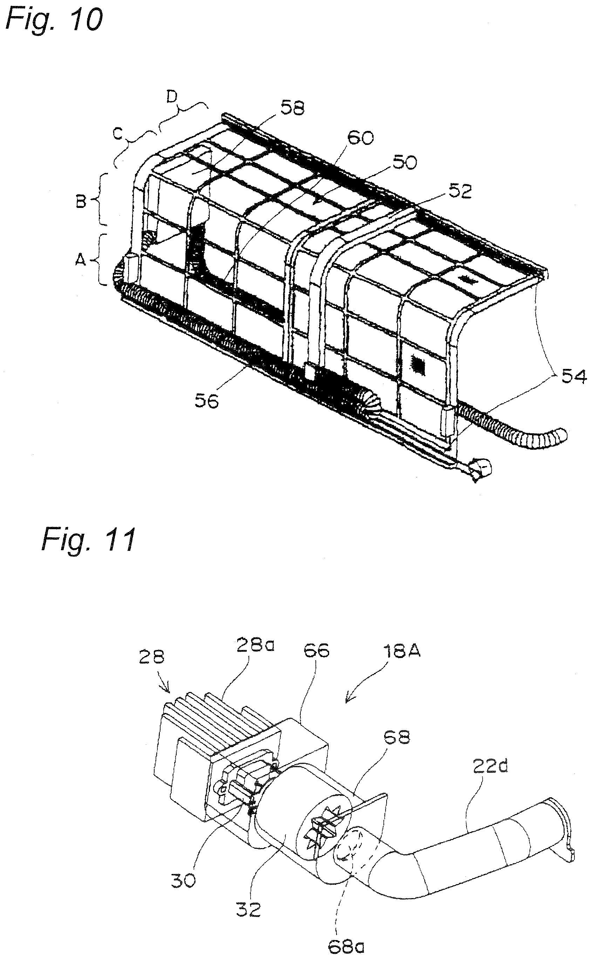

An air conditioner provided further with an automatic filter cleaning device having a suction device that removes dust adhering to the filter 5 by suction is explained hereinafter. The ventilation fan unit 16 is first explained with reference to Fig. 9. The ventilation fan unit 16 may be dedicated to ventilation or may also serve as the suction device in the indoor unit having the automatic filter cleaning device. Although the ventilation fan unit 16 as shown in Fig. 9 is incorporated into a suction device 58 of the automatic filter cleaning device on the side of the bypass passage 22 with respect to the partition wall 46c, the automatic filter cleaning device is simply explained with reference to Fig. 10, because it is already known. A detailed construction or an operating method of the automatic filter cleaning device is not particularly limited.

As shown in Fig. 10, an automatic filter cleaning device 50 includes a suction nozzle 52 that can slide along the surface of the filter 5. The suction nozzle 52 can horizontally smoothly move along a pair of guide rails 54 respectively disposed at upper and lower ends of the filter 5 with an extremely small gap kept between the suction nozzle 52 and the filter 5 to thereby remove dust adhering to the filter 5 by suction. One end of a flexible suction duct 56 is connected to the suction nozzle 52, and the other end of the suction duct 56 is connected to the suction device 58 having a varying suction capability. A discharge duct 60 is similarly connected to the suction device 58 and led outside.

A belt (not shown) is vertically wound around the suction nozzle 52 so as to be slidable along the suction nozzle 52. The suction nozzle 52 has a nozzle opening in the form of a slit defined in a wall confronting the filter 5. The nozzle opening has a vertical length substantially equal to the vertical length of the filter 5. On the other hand, the belt has a suction opening in the form of a slit having a vertical length equal to, for example, a quarter of the vertical length of the filter 5.

The automatic filter cleaning device 50 of the above-described construction sequentially cleans respective cleaning areas A, B, C, and D of the filter 5 as occasion demands. When the cleaning area A is cleaned, the belt is vertically driven so that the suction opening thereof may be fixed to the position of the cleaning area A. The suction nozzle 52 is subsequently horizontally driven from a right end to a left end of the filter 5 while sucking dust together with air through the suction opening, thereby cleaning the horizontally extending cleaning area A of the filter 5.

Next, the belt is vertically driven so that the suction opening thereof may be fixed to the position of the cleaning area B. Under this condition, the suction nozzle 52 is horizontally driven from the left end to the right end of the filter 5 while sucking dust together with air through the suction opening, thereby cleaning the horizontally extending cleaning area B of the filter 5. The cleaning areas C and D of the filter 5 are similarly cleaned by suction.

Dust adhering to the filter 5 and sucked by the suction nozzle 52 is discharged outside the room through the suction duct 56, the suction device 58, and the discharge duct 60.

With further reference to Fig. 9, a suction passage of the suction device 58 has an opening 62 defined therein and a damper 64 for opening and closing the opening 62. The ventilation fan unit 16 is used for ventilation when the damper 64 has opened the opening 62, while when the filter 5 is cleaned by suction, the damper 64 closes the opening 62 so that the ventilation fan unit 16 may be used to suck dust through the suction opening in the belt. That is, the suction cleaning function and the ventilation function are both realized using only the suction device 58.

It is to be noted that although the discharge duct 60 is not shown in Fig. 9, the discharge duct 60 is connected to a discharge port 58a defined in the suction device 58.

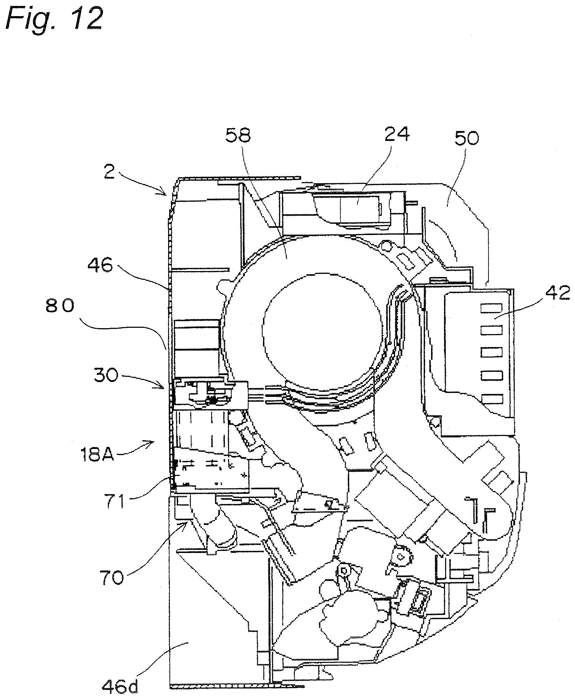

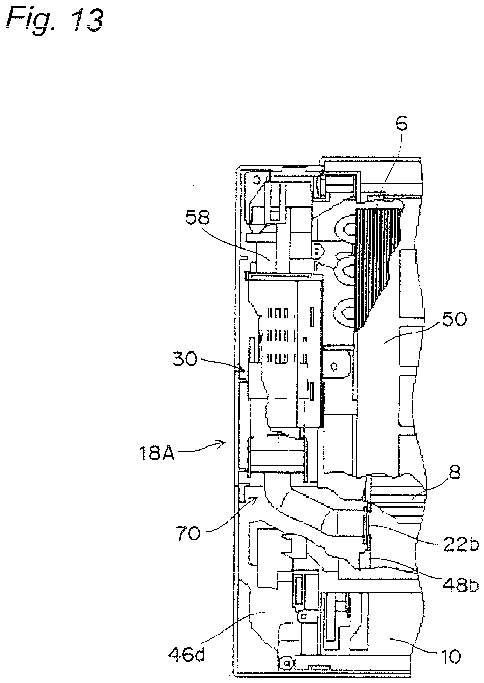

Fig. 11 depicts an electrostatic atomizing device 18A having no casing. This electrostatic atomizing device 18A is incorporated in the indoor unit body 2, as shown in Figs. 12 and 13. The electrostatic atomizing device 18A is placed at a position overlapping with the ventilation fan unit 16 as viewed from above or from the front of the indoor unit, and such position is a position in proximity to the opening 62 in the ventilation fan unit 16 and the damper 64, through which position air induced by the ventilation fan unit 16 flows.

More specifically, in the electrostatic atomizing device 18A of Fig. 11, the electrostatic atomizing unit 30 having the radiating portion 28 is integrated with the silencer 32, and the electrostatic atomizing unit 30 excluding the radiating portion 28 and the silencer 32 are accommodated within respective housings (a unit housing 66 and a silencer housing 68). The silencer housing 68 has an opening 68a defined therein, to which one end of the bypass discharge pipe 22d is connected. The other end of the bypass discharge pipe 22d is connected to the main passage 20. As described above, the radiating portion 28 has a plurality of fins 28a with air gaps defined therebetween, and the fins 28a are arrayed so that the air gaps may be orientated substantially along an air flow induced by a negative pressure, i.e., the pressure of the main passage 20 that is lower than that of the bypass passage 22, or substantially along an air flow created by the suction of the ventilation fan unit 16.

In this case, a storage space 22e, which is separated from the main passage 20 by the partition wall 46c and formed between the partition wall 46c and a left side wall of the main body cover, and in which the ventilation fan unit 16, the electrostatic atomizing device 18A, and the like are accommodated, acts in place of the bypass suction pipe 22c and the casing 34 both referred to above. The storage space 22e also accommodates the bypass discharge pipe 22d and acts as the bypass passage 22.

By this construction, air drawn into the main body 2 through the filter 5 is sucked into the storage space 22e through the bypass suction port 22a positioned downstream of the filter 5. Such air flows through the storage space 22e in a direction parallel to an air flow in the main passage 20 as viewed from the front of the main body 2 of the indoor unit. The air flowing through the storage space 22e cools the radiating portion 28 and is drawn into the electrostatic atomizing unit 30 through an opening or openings (not shown) defined in the unit housing 66.

The above-described construction causes a space formed around and juxtaposed with the ventilation fan unit 16 in an overlapping manner as viewed from above or from the front of the indoor unit to act as the bypass passage 22, and a reduction in the mounting space can be accomplished by making effective use of the storage space 22e for the ventilation fan unit 16, the electrostatic atomizing device 18A, and the like. Also, because the air gaps between the fins 28a are orientated substantially along the air flow, air flows efficiently through the air gaps, thereby making it possible to further efficiently cool Peltier elements 36.

In this construction, the high voltage transformer 24 is placed at an arbitrary position in the storage space 22e accommodating the ventilation fan unit 16, the electrostatic atomizing device 18A, and the like, and the bypass fan 26 is dispensed with.

As described above, the bypass passage 22 is separated from the main passage 20 merely by the partition wall 46c so that an air flow in the bypass passage 22 may become parallel to an air flow in the main passage 20 as viewed from the front of the main body 2 of the indoor unit, thus making it possible to easily provide the bypass passage 22 and reduce the number of component parts.

Further, the above-described construction enables a single filter 5 to be shared between the main passage 20 and the electrostatic atomizing device 18A.

It is to be noted here that an opening 46d may be formed in the vicinity of a lower portion of the frame 46 positioned rearwardly of the ventilation fan unit 16 so that pipes (not shown) for connecting the indoor unit and the outdoor unit may be installed through the opening 46d (see Fig. 9). The bypass suction port 22a referred to above is an opening defined in the partition wall 46c (side wall 46b of the frame) to suck air into the storage space 22e therethrough, and the bypass suction port 22a communicates with the outside of the indoor unit through the filter 5. However, the opening 46d defined in the lower portion of the frame 46 is an opening through which the storage space 22e communicates directly with the outside of the indoor unit to suck ambient air. In this case, the storage space 22e is a bypass passage that also bypasses the filter 5. Accordingly, air sucked into the electrostatic atomizing device 18A passes through the opening 46d and not through the filter 5 and, hence, another filter for the electrostatic atomizing device 18A may be provided as occasion demands. Even if the opening 46d is provided, the fact remains that the electrostatic atomizing device 18A is juxtaposed with the ventilation fan unit 16 in an overlapping manner as viewed from above or from the front of the indoor unit, and a reduction in the mounting space can be accordingly similarly accomplished by making effective use of the storage space 22e.

As described above, because the indoor fan 8 imparts a predetermined speed to an air flow on the side of the main passage 20 with respect to the bypass discharge port 22b, a pressure difference is created between respective sides of the partition wall 46c. That is, the pressure on the side of the main passage 20 becomes negative and, hence, even if the bypass fan 26 is not provided, the radiating portion 28 is cooled by air that is introduced from the storage space 22e, being the bypass passage, toward the main passage 20 through the bypass discharge pipe 22d, and the electrostatic mist generated by the electrostatic atomizing unit 30 is introduced into the main passage 20 and discharged into the room to be air conditioned. Also, because the radiating portion 28 is positioned in the vicinity of the opening 62 and the damper 64 and at a location where air flows before it is sucked through the opening 62, the radiating portion 28 is also cooled by the suction air into the ventilation fan unit 16.

In the construction referred to above, the main passage 20 and the storage space 22e being the bypass passage are separated by the partition wall 46c, and the electrostatic atomizing device 18A for generating the electrostatic mist is provided in the storage space 22e. Accordingly, the electrostatic atomizing device 18A is supplied with air that does not pass the heat exchanger 6 and is accordingly not controlled in temperature and humidity, and dew condensation on the whole Peltier elements 36 of the electrostatic atomizing unit 30 is effectively prevented during cooling, thereby enhancing safety, while the electrostatic mist can be positively generated even during heating, thus making it possible to steadily generate the electrostatic mist irrespective of the mode of operation of the air conditioner, i.e., at any time of year.

### Embodiment 2.

Another method of cooling the radiating portion 28 of the electrostatic atomizing device 18A shown in Fig. 11 is explained hereinafter.

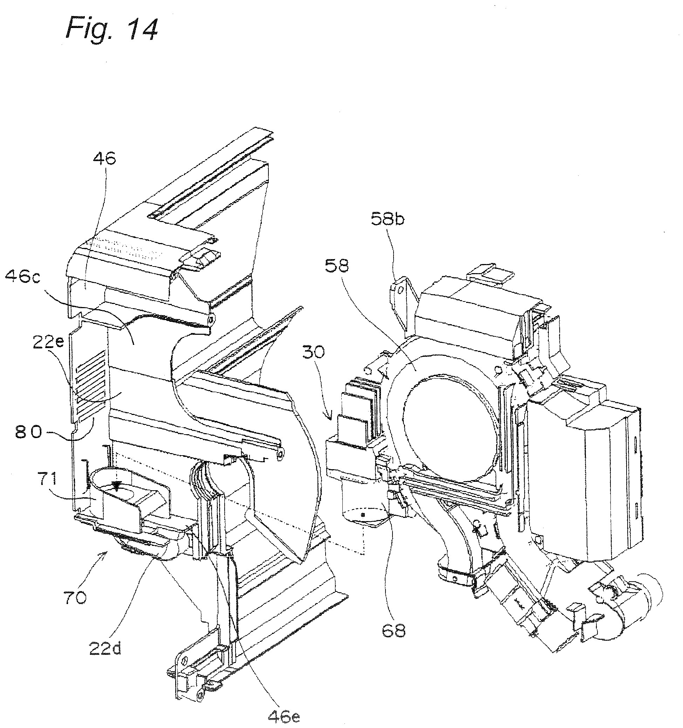

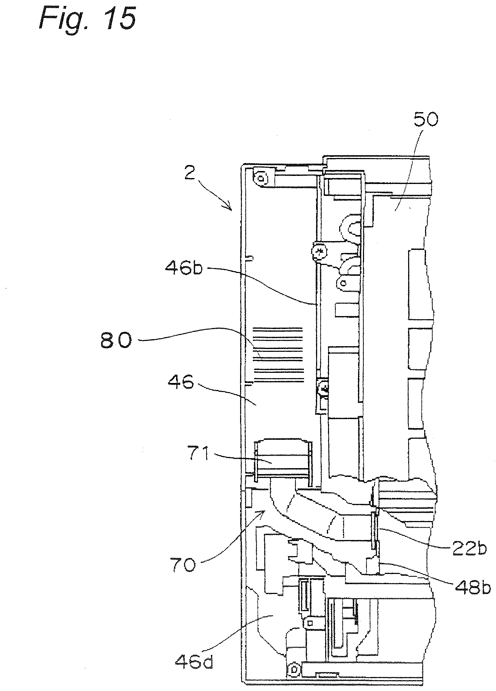

The radiating portion 28 of the electrostatic atomizing device 18A for cooling the Peltier elements 36 has a plurality of fins 28a with air gaps defined therebetween and, as shown in Figs. 11 to 14, the fins 28a are arrayed so as to extend vertically. As shown in Figs. 14 to 16, the rear wall 46a of the frame 46 defining the storage space 22e has a plurality of openings 80 each in the form of, for example, a slit leading to the outside of the indoor unit at a location confronting the fins 28a.

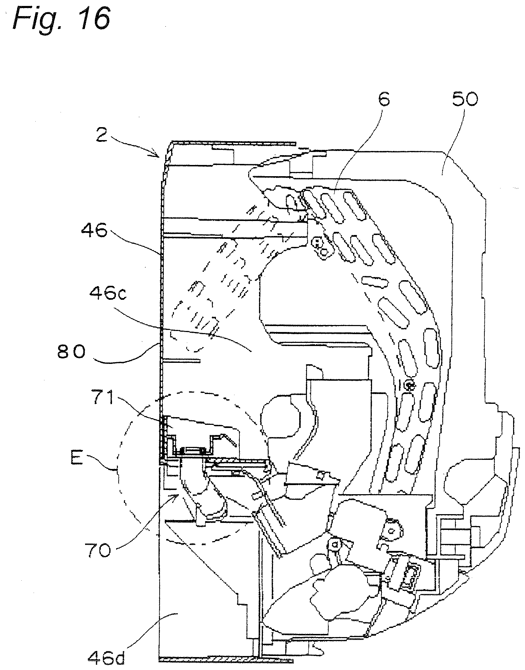

Further, as shown in Fig. 14, when the suction device 58 is incorporated into the frame 46, a gap or gaps (an opening or openings) are created in the partition wall 46c confronting the suction device 58 to communicate the main passage 20 and the bypass passage 22 with each other. In some cases, a slight gap is created between the side wall (left side wall) 46b of the frame 46 and the side wall (left side wall) 48b of the rear guider 48, both of which constitute the partition wall 46c. As a result, air inside the bypass passage 22 is drawn into the main passage 20 below the heat exchanger 6 as shown in Fig. 16 and upstream of the indoor fan 8 by the action of the negative pressure induced by the rotation of the indoor fan 8, thereby generating a new air flow inside the bypass passage 22, which in turn forcibly cools the electrostatic atomizing device 18A.

Because the openings 80 are formed in the rear wall 46a of the frame 46 confronting the air gaps between the fins 28a, air outside the indoor unit drawn into the bypass passage 22 passes smoothly through the air gaps between the fins 28a to efficiently cool the fins 28a, thus enhancing the radiation performance of the fins 28a and also enhancing the cooling effect of the Peltier elements 36.

In addition, the indoor fan 8 separated from the bypass passage 22 sucks air inside the bypass passage 22, which in turn induces entry of air from the openings 80. Such air results in a substantially forwardly directed flow because the gap below the heat exchanger 6 and the openings 80 are located on the same level in this embodiment. Accordingly, the air efficiently flows through the air gaps between the fins 28a that extend substantially along the air flow induced by the indoor fan 8, thus making it possible to further efficiently cool the Peltier elements 36.

Even if the gap below the heat exchanger 6 and the openings 80 are located on different levels and, hence, a vertical air flow would be created, if the fins 28a are oriented vertically and in the front-back direction, the air flow passes smoothly through the air gaps between the fins 28a to efficiently cool the fins 28a. In any event, the fins 28a have only to be oriented so that the air gaps therebetween may allow the air flow to pass smoothly therethrough.

Also, as shown in Fig. 11 or 14, the fins 28a of the radiating portion 28 are formed into a mound such that they are highest at a central portion of the radiating portion 28 confronting the Peltier elements 36 and become lower toward the outside. Accordingly, the centrally-located fins 28a effectively dissipate heat because they are brought into contact with fresh air from the side due to a difference in the height of the fins 28a, thus further enhancing the cooling effect of the Peltier elements 36.

Because the Peltier elements 36 are effectively cooled by the above-described construction, much dew condensation water can be obtained by increasing the voltage to be applied to the Peltier elements 36, thus making it possible to increase the amount of the electrostatic mist.

### Industrial Applicability

The air conditioner according to the present invention can effectively cool the Peltier elements to thereby positively generate an electrostatic mist and, hence, the air conditioner according to the present invention is particularly useful for air conditioners for home use to be mass-produced.

### Explanation of Reference Numerals

2 indoor unit body, 2a front suction opening, 2b upper suction opening,
4 front panel, 5 filter, 6 heat exchanger, 8 indoor fan,
10 discharge opening, 12 vertical air direction changing blade,
14 horizontal air direction changing blade, 16 ventilation fan unit,
18, 18A electrostatic atomizing device, 20 main passage,
22 bypass passage, 22a bypass suction port, 22b bypass discharge port,
22c bypass suction pipe, 22d bypass discharge pipe, 22e storage space,
24 high voltage transformer, 26 bypass fan, 28 radiating portion, 28a fin,
30 electrostatic atomizing unit, 32 silencer, 34 casing,
36 Peltier element, 36a radiating surface, 36b cooling surface,
38 discharge electrode, 40 opposing electrode, 42 controller,
44 Peltier drive power source, 46 frame, 46a rear wall, 46b side wall,
46c partition wall, 46d opening, 48 rear guider, 48a rear wall,
48b side wall, 50 automatic filter cleaning device, 52 suction nozzle,
54 guide rail, 56 suction duct, 58 suction device, 58a discharge port,
60 discharge duct, 62 opening, 64 damper, 66 unit housing,
68 silencer housing, 68a opening, 80 opening.

## Claims

1. An air conditioner having an indoor unit that includes a heat exchanger (6) and an indoor fan (8) operable to convey air heat exchanged by the heat exchanger (6), the indoor unit having a discharge opening (10) defined therein, through which the air conveyed by the indoor fan (8) is blown out, said air conditioner comprising:
a partition wall (46c) that partitions an inside of the indoor unit into first and second compartments with the heat exchanger (6) and the indoor fan (8) accommodated within the first compartment, the first compartment defining a main passage (20) through which the air conveyed by the indoor fan (8) is conveyed before the air is blown out through the discharge opening (10), the second compartment acting as a bypass passage (22) for bypassing the heat exchanger (6) and the indoor fan (8), the partition wall (46c) having an opening defined therein through which the first and second compartments communicate with each other; and
an electrostatic atomizing unit (30) accommodated within the second compartment and operable to generate dew condensation using moisture contained in air, then generate an electrostatic mist by high voltage discharge, and discharge the electrostatic mist, said electrostatic atomizing unit (30) comprising:
a Peltier element (36) operable to generate dew condensation water by cooling;
a discharge electrode (38) operable to cause the high voltage discharge to generate the electrostatic mist from the dew condensation water; and
a radiating portion (28) operable to dissipate heat from the Peltier element (36), the radiating portion (28) comprising a plurality of fins (28a) with air gaps defined therebetween;
wherein operation of the indoor fan (8) generates a negative pressure inside the main passage (20) to thereby introduce air inside the second compartment into the first compartment through the opening in the partition wall (46c); and
wherein the air gaps between the plurality of fins (28a) are formed substantially in a direction in which the air introduced from the second compartment into the first compartment flows.

2. The air conditioner according to claim 1, wherein the opening in the partition wall (46c) is positioned adjacent the discharge opening (10), and operation of the indoor fan (8) renders the main passage (20) to be a negative pressure portion of a pressure lower than that of the second compartment.

3. The air conditioner according to claim 1, wherein the opening in the partition wall (46c) is positioned upstream of the indoor fan (8), and operation of the indoor fan (8) renders the main passage (20) to be a negative pressure portion of a pressure lower than that of the second compartment.

4. The air conditioner according to any one of claims 1 to 3, wherein the fins (28a) of the radiating portion (28) are highest at a central portion confronting the Peltier element (36) and become lower toward outside.

5. The air conditioner according to any one of claims 1 to 4, wherein the indoor unit has an opening defined in a portion thereof delimiting the second compartment for communication with an outside of the indoor unit such that the opening confronts the air gaps between the fins (28a), and air outside the indoor unit is introduced into the second compartment by a suction force of the indoor fan (8) to flow through the air gaps.

## Patentansprüche

1. Klimaanlage mit einer innenliegenden Einheit, die einen Wärmetauscher (6) und einen innenliegenden Lüfter (8) aufweist, der so angetrieben werden kann, dass dieser die von dem Wärmetauscher (6) ausgetauschte Luftwärme transportiert, wobei die innenliegende Einheit eine darin definierte Austrittsöffnung (10) aufweist, durch die die vom innenliegenden Lüfter (8) geförderte Luft ausgeblasen wird, die Klimaanlage enthaltend:
eine Trennwand (46c), die ein Inneres der innenliegenden Einheit in erste und zweite Kammern unterteilt, wobei der Wärmetauscher (6) und der innenliegende lüfter (8) in der ersten Kammer untergebracht sind, wobei die erste Kammer einen Hauptkanal (20) definiert, durch den die von dem innenliegenden Lüfter (8) geförderte Luft transportiert wird, bevor die Luft durch die Austrittsöffnung (10) ausgeblasen wird, wobei die zweite Kammer als Bypass-Kanal (22) zum Umgehen des Wärmtauschers (6) und des innenliegenden Lüfters (8) dient, wobei die Trennwand (46c) eine darin definierte Öffnung aufweist, durch die die erste und zweite Kammer miteinander verbunden sind; und
eine elektrostatische Zerstäubungseinheit (30), die in der zweiten Kammer untergebracht ist und betreibbar ist, um Taukondensation mit Hilfe der in der Luft enthaltenden Feuchtigkeit zu erzeugen, um dann einen elektrostatischen Nebel durch Hochspannungsentladung zu erzeugen, und den elektrostatischen Nebel auszutragen, wobei die elektrostatische Zerstäubungseinheit (30) enthält:
ein Peltier-Element (36), das zum Erzeugen von Taukondensationswasser durch Kühlung betreibbar ist;
eine Entladungselektrode (38), die betreibbar ist, um die Hochspannungsentladung dazu zu veranlassen, den elektrostatischen Nebel aus dem Taukondensationswasser zu erzeugen; und
einen Strahlungsabschnitt (28), der betreibbar ist, um Wärme vom dem Peltier-Element (36) abzuleiten, wobei der Strahlungsabschnitt (28) eine Mehrzahl an Lamellen (28a) mit dazwischen definierten Luftspalten enthält;
wobei der Betrieb des innenliegenden Lüfters (8) einen Unterdruck innerhalb des Hauptkanals (20) erzeugt, um dadurch Luft innerhalb der zweiten Kammer durch die Öffnung in der Trennwand (46c) in die erste Kammer einzuführen; und
wobei die Luftspalte zwischen der Mehrzahl an Lamellen (28a) im Wesentlichen in eine Richtung ausgebildet sind, in die die von der zweiten Kammer in die erste Kammer eingeleitete Luft strömt.

2. Klimaanlage nach Anspruch 1, wobei die Öffnung in der Trennwand (46c) neben der Austrittsöffnung (10) angeordnet ist, und der Betrieb des innenliegenden Lüfters (8) den Hauptkanal (20) zu einem Unterdruckabschnitt macht, dessen Druck niedriger ist als der der zweiten Kammer.

3. Klimaanlage nach Anspruch 1, wobei die Öffnung in der Trennwand (46c) stromaufwärts des innenliegenden Lüfters (8) angeordnet ist, und der Betrieb des innenliegenden Lüfters (8) den Hauptkanal (20) zu einem Unterdruckabschnitt macht, dessen Druck niedriger ist als der der zweiten Kammer.

4. Klimaanlage nach einem der Ansprüche 1 bis 3, wobei die Lamellen (28a) des Strahlungsabschnitts (28) an einem zentralen Abschnitt, der dem Peltier-Element (36) gegenüberliegt, am höchsten sind und nach außen hin niedriger werden.

5. Klimaanlage nach einem der Ansprüche 1 bis 4, wobei die innenliegende Einheit eine Öffnung aufweist, die in einem Abschnitt davon definiert ist, die die Verbindung der zweiten Kammer mit einer Außenseite der innenliegenden Einheit so begrenzt, dass die Öffnung den Luftspalten zwischen den Lamellen (28a) gegenüberliegt, und Luft außerhalb der innenliegenden Einheit durch eine Saugkraft des innenliegenden Lüfters (8) in die zweite Kammer eingeleitet wird, um durch die Luftspalte zu strömen.

## Revendications

1. Climatiseur ayant une unité intérieure qui comprend un échangeur thermique (6) et un ventilateur intérieur (8) pouvant fonctionner pour transporter la chaleur d'air échangée par l'échangeur thermique (6), l'unité intérieure ayant une ouverture de décharge (10) définie dans celle-ci, à travers laquelle l'air transporté par le ventilateur intérieur (8) est évacué par soufflage, ledit climatiseur comprenant :
une paroi de séparation (46c) qui sépare l'intérieur de l'unité intérieure en des premier et second compartiments avec l'échangeur thermique (6) et le ventilateur intérieur (8) logés à l'intérieur du premier compartiment, le premier compartiment définissant un passage principal (20) à travers lequel l'air transporté par le ventilateur intérieur (8) est transporté avant que l'air ne soit évacué par soufflage à travers l'ouverture de décharge (10), le second compartiment servant de passage de dérivation (22) pour contourner l'échangeur thermique (6) et le ventilateur intérieur (8), la paroi de séparation (46c) ayant une ouverture définie dans celle-ci à travers laquelle les premier et second compartiments communiquent l'un avec l'autre ; et
une unité d'atomisation électrostatique (30) logée à l'intérieur du second compartiment et pouvant fonctionner pour générer une condensation de rosée à l'aide de l'humidité contenue dans l'air, puis générer un brouillard électrostatique par décharge à haute tension, et décharger le brouillard électrostatique, ladite unité d'atomisation électrostatique (30) comprenant :
un élément Peltier (36) pouvant fonctionner pour générer de l'eau de condensation de rosée par refroidissement ;
une électrode de décharge (38) pouvant fonctionner pour amener la décharge à haute tension à générer le brouillard électrostatique à partir de l'eau de condensation de rosée ; et
une partie rayonnante (28) pouvant fonctionner pour dissiper la chaleur provenant de l'élément Peltier (36), la partie rayonnante (28) comprenant une pluralité d'ailettes (28a) avec des espaces d'air définis entre celles-ci ;
dans lequel le fonctionnement du ventilateur intérieur (8) génère une pression négative à l'intérieur du passage principal (20) pour ainsi introduire de l'air à l'intérieur du second compartiment dans le premier compartiment à travers l'ouverture dans la paroi de séparation (46c) ; et
dans lequel les espaces d'air entre la pluralité d'ailettes (28a) sont formés sensiblement dans une direction dans laquelle l'air introduit à partir du second compartiment dans le premier compartiment s'écoule.

2. Climatiseur selon la revendication 1, dans lequel l'ouverture dans la paroi de séparation (46c) est positionnée de manière adjacente à l'ouverture de décharge (10), et le fonctionnement du ventilateur intérieur (8) amène le passage principal (20) à être une partie de pression négative d'une pression inférieure à celle du second compartiment.

3. Climatiseur selon la revendication 1, dans lequel l'ouverture dans la paroi de séparation (46c) est positionnée en amont du ventilateur intérieur (8), et le fonctionnement du ventilateur intérieur (8) amène le passage principal (20) à être une partie de pression négative d'une pression inférieure à celle du second compartiment.

4. Climatiseur selon l'une quelconque des revendications 1 à 3, dans lequel les ailettes (28a) de la partie rayonnante (28) sont les plus hautes au niveau d'une partie centrale faisant face à l'élément Peltier (36) et deviennent inférieures vers l'extérieur.

5. Climatiseur selon l'une quelconque des revendications 1 à 4, dans lequel l'unité intérieure a une ouverture définie dans une partie de celle-ci délimitant le second compartiment pour une communication avec un extérieur de l'unité intérieure de telle sorte que l'ouverture fait face aux espaces d'air entre les ailettes (28a), et de l'air à l'extérieur de l'unité intérieure est introduit dans le second compartiment par une force d'aspiration du ventilateur intérieur (8) pour s'écouler à travers les espaces d'air.
